# EUROPEAN PATENT SPECIFICATION

(11) **EP 3 302 546 B1**
(45) Date of publication and mention of the grant of the patent: **25.10.2023**
(21) Application number: 16727375.4
(22) Date of filing: 26.05.2016
(51) Int. Cl.: A61K 39/35, A61K 39/00

(54) **ALLERGY-SPECIFIC IMMUNOTHERAPY COMPOSITIONS FOR USE IN THE TREATMENT OF HOUSE-DUST MITE ALLERGY**
ALLERGIESPEZIFISCHE IMMUNTHERAPIEZUSAMMENSETZUNGEN ZUR VERWENDUNG BEI DER BEHANDLUNG VON HAUSSTAUBMILBENALLERGIE
COMPOSITIONS POUR IMMUNOTHÉRAPIE SPÉCIFIQUE DE L'ALLERGIE À UTILISER DANS LE TRAITEMENT DE L'ALLERGIE AUX ACARIENS

(30) Priority: 29.05.2015 NL 2014882
(43) Date of publication of application: 11.04.2018
(73) Proprietor: Citeq B.V., 9726 GN Groningen (NL)
(72) Inventor: EUVERINK, Gerrit Jan Willem, 9752 NE Haren (NL); HESSE, Laura, 9321 GH Peize (NL); NAWIJN, Martijn Christiaan, 9616 TL Scharmer (NL); VAN DER GRAAF, Adrianus Cornelis, 9712 BP Groningen (NL)
(74) Representative: Hoyng Rokh Monegier B.V.
(86) International application number: PCT/EP2016/061947
(87) International publication number: WO 2016/193126

(56) References cited:
- WO-A1-00/50044
- WO-A1-2011/151449
- WO-A2-2014/195803
- KUAN-WEI CHEN ET AL: "Hypoallergenic Der p 1/Der p 2 combination vaccines for immunotherapy of house dust mite allergy", JOURNAL OF ALLERGY AND CLINICAL IMMUNOLOGY, ELSEVIER, AMSTERDAM, NL, vol. 130, no. 2, 23 May 2012 (2012-05-23), pages 435-443.e4, XP028431297, ISSN: 0091-6749, DOI: 10.1016/J.JACI.2012.05.035 [retrieved on 2012-06-02]
- KIM NACKSUNG ET AL: "Protective effect of the DNA vaccine encoding the major house dust mite allergens on allergic inflammation in the murine model of house dust mite allergy", CLINICAL AND MOLECULAR ALLERGY, BIOMED CENTRAL LTD., LONDEN, GB, vol. 4, no. 1, 20 February 2006 (2006-02-20), page 4, XP021018406, ISSN: 1476-7961, DOI: 10.1186/1476-7961-4-4
- OLIVER PFAAR ET AL: "Mite-Allergic Rhinitis: How to Evaluate Clinical Efficacy in Allergen-Specific Immunotherapy Trials?", CURRENT TREATMENT OPTIONS IN ALLERGY, vol. 2, no. 1, 15 January 2015 (2015-01-15), pages 1-9, XP055204446, DOI: 10.1007/s40521-014-0040-y
- YUBAO CUI: "Structural biology of mite allergens", MOLECULAR BIOLOGY REPORTS, vol. 40, no. 1, 1 January 2013 (2013-01-01), pages 681-686, XP055204631, ISSN: 0301-4851, DOI: 10.1007/s11033-012-2108-8
- Chen-Xia Xu ET AL: "Efficacy of Sublingual Immunotherapy with Dermatophagoides farinae Extract in Monosensitized and Polysensitized Patients with Allergic Rhinitis: Clinical Observation and Analysis", BioMed Research International, vol. 2015, 1 January 2015 (2015-01-01), pages 1-5, XP055669395, ISSN: 2314-6133, DOI: 10.1155/2015/187620
- Chien-Hui Chien ET AL: "Single allergen-induced oral tolerance inhibits airway inflammation in conjugated allergen immunized mice", Journal of Allergy and Clinical Immunology, vol. 136, no. 4, 1 October 2015 (2015-10-01), pages 1108-1110.e2, XP055669399, AMSTERDAM, NL ISSN: 0091-6749, DOI: 10.1016/j.jaci.2015.03.046

## Description

### FIELD OF THE INVENTION

The present invention relates to compositions comprising house dust mite allergens for use in the treatment of house-dust mite allergy.

### BACKGROUND OF THE INVENTION

Allergic diseases, including type I-IV hypersensitivity reactions, are thought to affect between 25 to 40 percent of the population in developed countries. Allergic reactions such as allergic rhinitis (hay fever), asthma, and hives (urticarial) are an oversensitivity of the immune system to a respective, otherwise harmless, allergen, characterized by an increased ability of B-lymphocytes to produce immunoglobulin E (IgE), increased presence and activity of eosinophiles and elevated levels of pro-inflammatory cytokines IL-4, IL-5 and IL-13. In allergic asthma an allergic reaction is further characterized by increased airway hyperresponsiveness.

As is generally known, an allergen is a type of antigen that produces an abnormally vigorous immune response in which the immune system fights off a perceived threat that would otherwise be harmless to the body. An antigen is a molecule capable of inducing an immune response on the part of the host organism.

House-dust mites (HDM) represent one of the most important allergen sources; more than 50% of allergic patients are sensitized to mite allergens. The allergens of *Dermatophagoides pteronyssinus* (Der p) comprise more then 30 proteins and glycoproteins, which show cross-reactivity to allergens from other mite species, for example to those of *Dermatophagoides farinae* (Derf). Among the 19 described groups of HDM allergens, group 1 and 2 (Der p1, a cysteine protease, and Der p2, a transporter protein) represent the most important allergens. Between 50% and 100% of allergic individuals have IgE antibodies specific against these two allergens. Der f 1, one of the major allergens of *Dermatophagoides farinae,* is also a cysteine protease and shows 82% homology to Der p 1 (Deb R. et al. Journal of Biological Chemistry, 2007, 282, 36808-36819).

Allergen-specific immunotherapy (SIT) represents a disease-modifying approach with long-lasting effects in allergic patients, which is based on the administration of increasing doses of the disease-eliciting allergens. SIT protocols generally involve weekly administrations during a build-up phase, followed by monthly maintenance administration for a period of 2-5 years. Besides these long-term SIT treatment regimens accelerated, or rush, immunotherapy is performed wherein increasing doses of allergen are administered every few hours (Schiavino et al. Ann Allergy Asthma Immunol. 2004; 92(4):409-13.).

SIT treatment is geared towards the induction of allergen-specific IgG antibodies, which prevent allergic patients' IgE binding and hence inhibit IgE mediated effector cell degranulation and restore immune regulation by promoting the development of Thelper-1 (Th-1) or T regulatory (Tr) cells that are able to down-regulate the allergy-promoting Th-2 effector cells. The efficacy of SIT treatment varies between allergic disorders, with good efficacy for venom allergies and rhinitis, but poor efficacy for allergic asthma, and food allergy.

Current SIT treatment regimens routinely employ a crude extract of the allergen for subcutaneous injections or sublingual formulations. While the crude extract represents the full array of major allergens that a patient can be sensitized to, it also contains varying amounts of immunostimulatory components such as chitins, β-glucans as well as endotoxins that may elicit unwanted effects upon administration as well as limit clinical efficacy of SIT. As a result, administration of high doses of the crude allergen extracts has resulted in safety concerns, in particular with regards to the potential risk in triggering life-threatening, immediate IgE-mediated anaphylactic reactions.

To improve the safety of SIT, there is a need to develop modified allergens suitable for immunotherapy against house dust mite allergy.

It can furthermore be noted, that the crude extract of cultured house dust mites may show largely varying compositions, depending on whether the mites bodies themselves are mainly harvested, or their excrements, or both.

A review on house dust mite allergy and associated immunotherapy for asthma is published by Liu et al. Immunome Research 2014; 11:1-6, in which it is concluded that new approaches are still needed, despite many decades of research.

Der p1 and Der p2 proteins are major allergens produced by house dust mite. Der p1 has been isolated in 1980 (Chapman et al. J. of Immunology 1980; 125:587-592). Der p2 was described at least in 1986 (Yasueda et al. Int. Archs. Allergy appl. Immun. 1986; 81:214-223). Others have been described e.g. in European patent application EP1612219 and at least 20 protein based allergens are thought to be present in house dust mite.

Many efforts have been made to engineer recombinant Der p1 and Der p2 proteins. Der p1 is a cysteine protease whose IgE- and IgG-eliciting activity is dependent on its cysteine protease activity. A recombinant Der p1 that is successful in SIT has not yet been reported, despite for example WO92/04445 or US2007/122423. Recombinant Der p2 allergens structurally and immunologically resembling the natural allergens have been produced and used in experiments.

Chen et al. in J. Allergy Clin Immunol (2012) 130:435-443 describesa recombinant combination vaccine consisting of reassembled Der p1 and Der p2 fragments.

Kim et al. in Clin & Molecular Allergy (2006) 4:1-9 describes the use of a DNA vaccine, which DNA is supposed to encode for major house dust mite allergens, to achieve a protective effect in BALB/c mice. WO00/50044 suggests to apply gene therapy with DNA that encodes for T-cell epitopes to the house dust mite Der p.

A hybrid molecule disclosed in Asturias et al. Clinical & Experimental Allergy 2009; 39:1088-1098 consisting of a truncated Der p2 and Der p1 was shown to exhibit reduced reactivity with patients' IgE, but it has not been unambiguously demonstrated that this molecule was able to induce Der p1 specific IgG antibodies *in vivo.* A similar disclosure can be found in WO2009/118642 describing hybrid proteins composed of fragments of allergens Der p1 and Der p2, that were engineered to reduce their allergenic activity and thus therapy-induced side-effects. Overall, the mean inhibition of HDM allergic patients' IgE by IgG antibodies in response to a mix of recombinant Der p1 and Der p2 was not higher than a 20% mean inhibition.

Other approaches to find suitable compositions with allergens are described in EP2727934, WO2007/123488, WO2014/195803 and WO2008/092902.

WO2011/151449 describes the use of whole body and feacal extracts and the adjustment of the ratio of for example Der p1 and Der p2 allergens by mixing these extracts to achieve a certain ratio of Der p1 and Der p2 in order to lower the variability of whole mite extracts. It may be noted that extracted mite bodies comprise over 30 proteins and a substantial amount of non-protein materials, originating from the mite body or faeces.

Despite all these efforts, the only commonly used allergen for treating HDM allergy is HDM extract.

The object of the present invention is to provide an allergen composition suitable for treating HDM allergy, which is more effective than the currently used HDM extract.

### SUMMARY OF THE INVENTION

### The invention provides for a pharmaceutical composition for use, according to claim 1

The purified nDerp1 and nDerp2 or purified nDerf1 and nDerf2, can be used in a method of allergen-specific immunotherapy against HDM allergy and may be administered to a mammal sensitized with HDM allergens without eliciting anaphylactic shock. In view of the known high cross-reactivity, the HDM is not limited to *Der* p., but can be *Der* f*.* or other types of HDM.

More specifically, the invention relates to a SIT method involving administering the composition of the present invention to accustom the body to HDM allergens and thereby induce specific long-term tolerance and reduce allergic symptoms.

The mammal preferably is a human. The allergic reaction is most important to be desensitized for mammals, showing symptoms of asthma, rhinitis or dermatitis.

The composition is administered repeatedly according to a specific immunotherapy regimen (a SIT protocol) one to several times daily, weekly, monthly and/or yearly, with increasing dosages during the initial phase of the treatment.

Administration of allergens generally is performed systemically to allow the body to develop IgG antibodies.

Such administration preferably comprises parenteral, transdermal, intradermal, or oral administration or combinations thereof. A preferred parenteral route is via subcutaneous injection. Another preferred route is transdermal, preferably sublingual. In the more preferred embodiment the allergen composition is administered subcutaneously or transdermal, and most preferred subcutaneous.

The present invention provides the possibility of treating allergy to HDM using the two major HDM allergens in purified, natural form, wherein the treatment leads to effective desensitization to HDM in general.

Unexpectedly, the treatment with a composition comprising purified nDerp1 and purified nDerp2 was more effective in eliciting desensitization, than treatment with crude extract of HDM in an animal model of allergic asthma.

### BRIEF DESCRIPTION OF THE DRAWINGS

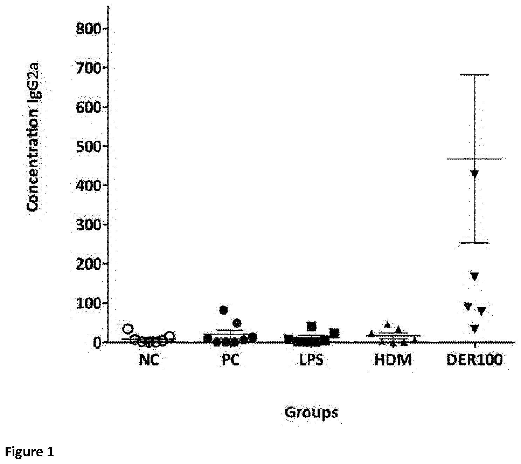

Figure 1 shows HDM specific IgE and allergen neutralizing IgG1 and IgG2 in house dust mite sensitized mice, before and after SCIT treatment with 1) crude HDM extract (HDM, upward triangles), 2) purified nDer p1 and purified nDer p2 (DER100 downward triangles), and 3) LPS (squares) and 4)PBS controls (dots). * = p<0.05 in Mann Whitney U test.

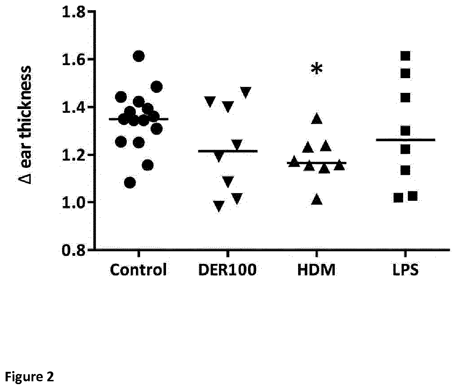

Figure 2 shows an ear swelling response expressed in fold change of ear thickness, as a marker for early phase hypersensitivity, after intradermal HDM injection in HDM sensitized mice, SCIT-treated with 1) PBS as a control (dots) 2) purified nDerp1 and nDerp2 (DER100, downward triangles) 3) crude HDM extract (upward triangles) 4) Lipopolysaccharides (LPS, squares). * = p<0.05 in Mann Whitney U test.

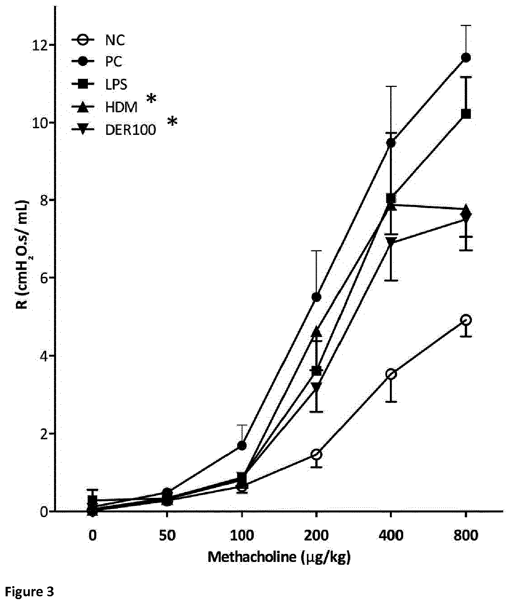

Figure 3 shows airway resistance (in cmH₂O.s/ml) in response to a dose-range of methacholine in HDM sensitized mice after HDM challenges. Figure 3 shows airway resistance following treatment with purified nDerp1 and nDerp2 (DER100, downward triangles), HDM extract (upward triangles), LPS (black squares), SCIT-PBS (positive asthma controls, PC, black dots) or after PBS challenge (negative controls for asthma manifestations, NC, open dots). * = p<0.05 in GEE analysis.

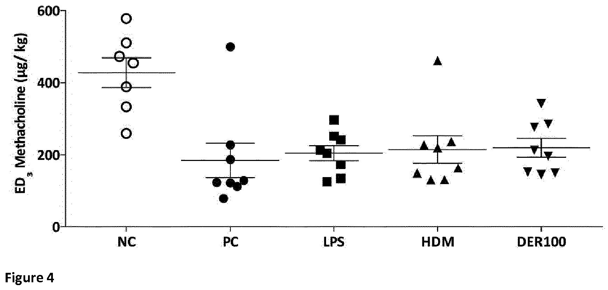

Figure 4 shows the estimated dose of methacholine (in µg methacholine per kg bodyweight) needed for an airway resistance of 3 cmH₂O^{∗}s/ml (ED3) in HDM sensitized mice after HDM challenges following SCIT treatment. Figure 4 shows the ED3 after SCIT treatment with HDM extract (HDM, upward triangles), purified nDerp1 and nDerp2 protein (DER100, downward triangles), LPS (squares), SCIT-PBS (PC, black dots) compared to PBS challenge (NC, open dots). * = p<0.05 in Mann Whitney U test.

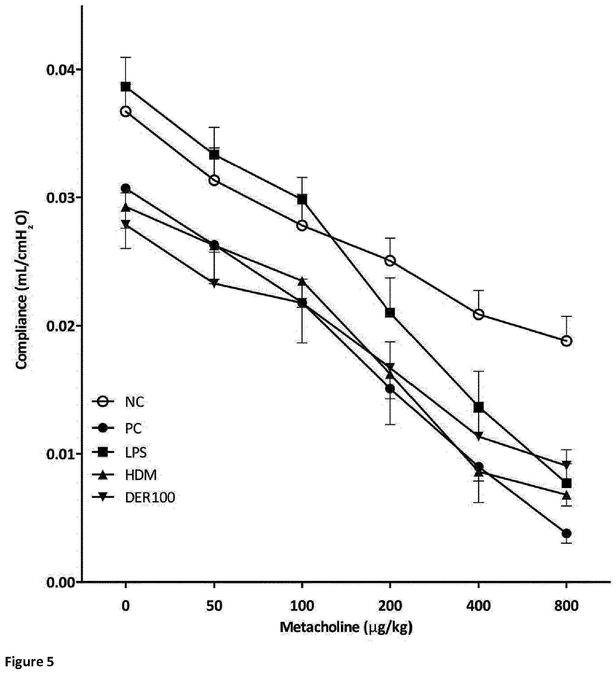

Figure 5 shows the lung compliance (in mL/cmH₂O) in HDM sensitized mice after HDM challenges following SCIT treatment. Figure 5 shows the lung compliance after SCIT treatment with HDM extract (HDM, upward triangles), purified nDerp1 and nDerp2 protein (DER100, downward triangles), LPS (squares), SCIT-PBS (PC, black dots) compared to PBS challenge (NC, open dots). * = p<0.05 in GEE analysis.

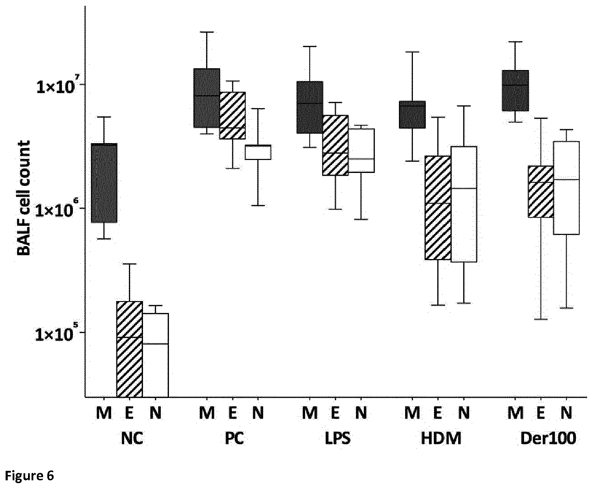

Figure 6 shows the absolute number of mononuclear cells (M), eosinophilic granulocytes (E), neutrophilic granulocytes (N) in broncho-alveolar lavage fluid as marker of airway inflammation after SCIT treatment with HDM extract (HDM, upward triangles), purified nDerp1 and nDerp2 protein (DER100, downward triangles), LPS (squares), SCIT-PBS (PC, black dots) compared to PBS challenge (NC, open dots). * = p<0.05 in Mann Whitney U test.

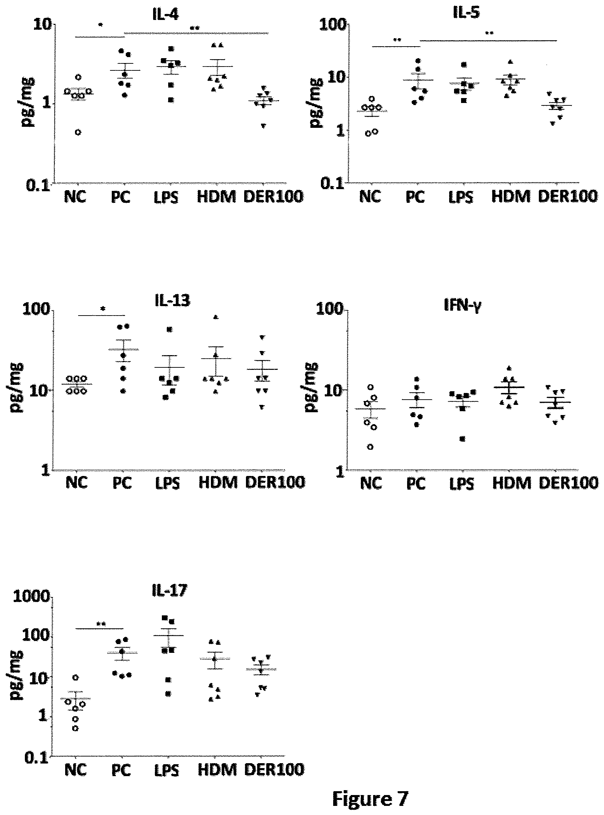

Figure 7 shows levels of T cell cytokines IL-4, IL-5, IL-13, IFN-γ and IL-17 in lung tissue (in pg cytokine per µg lung tissue protein) of HDM sensitized mice following SCIT treatment with HDM extract (HDM, upward triangles), purified nDerp1 and nDerp2 protein (DER100, downward triangles), LPS (black squares), SCIT-PBS (PC, dots) compared to PBS challenge (NC, open dots). Levels of each cytokine are shown in pg/pg lung tissue. * = p<0.05 in Mann Whitney U test.

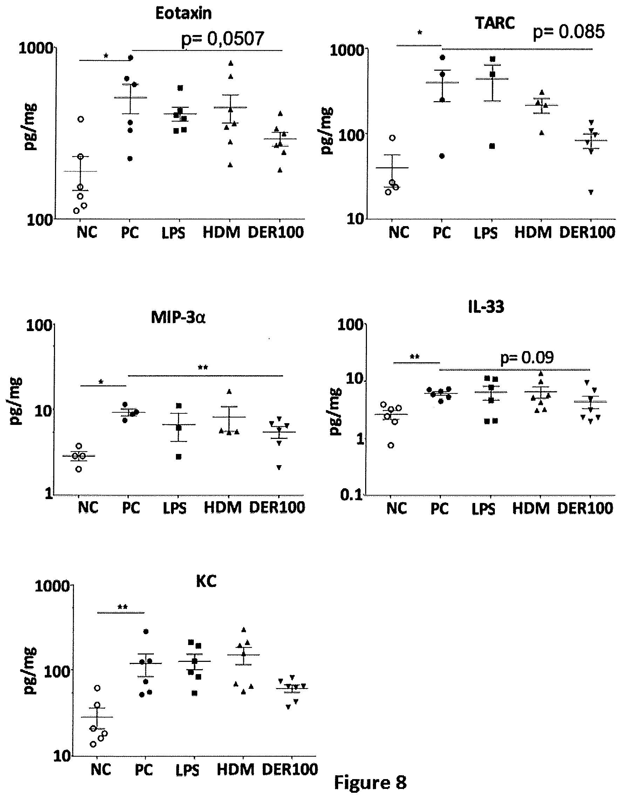

Figure 8 shows levels of chemokines and alarmins, eotaxin, TARC, CCL20, IL-33 and KC in lung tissue (in pg cytokine per µg lung tissue protein) of HDM sensitized mice following SCIT treatment with HDM extract (HDM, upward triangles), purified nDerp1 and nDerp2 protein (DER100, downward triangles), LPS (squares), SCIT-PBS (PC, black dots) compared to PBS challenge (NC, open dots). Levels of each cytokine are shown in pg/pg lung tissue. * = p<0.05 in Mann Whitney U test.

### DETAILED DESCRIPTION OF THE INVENTION

### Definitions

The term "Der p" means the house dust mite species called *Dermaphagoides pteronyssinus.* The term "Der p1"means the Der p allergen belonging to the group 1 according to the nomenclature of the Allergen Nomenclature Sub-committee, which operates under the auspices of the International Union of Immunological societies (IUIS) and the World Health Organization (WHO). The term "Der p2" means the Der p allergen belonging to group 2 according to the nomenclature of the Allergen Nomenclature Sub-committee. The term "Der f" means the house dust mite species called *Dermaphagoides farinae.* The term "Der f1"means the Der f allergen belonging to the group 1 and "Der f2"means the Der f allergen belonging to the group 2.

The term "natural" means the chemical structure of the protein is identical to the natural occurring protein in its primary, secondary and tertiary structure. Natural Der p1 (nDerp1) and natural Der p2 (nDerp2) as well as natural Der f1 and natural Der f2 are preferably isolated from house dust mites. However, it is known that Der p2 which fully reflects Der p2 isolated from house dust mite can be made via recombinant techniques. In several embodiments describing the invention in the 'detailed description of the invention' as well the examples, nDerp1 and nDerp2 are specifically mentioned. However, the invention is not limited thereto, and also encompasses nDerf1 and nDerf2. However, in a preferred embodiment, the invention relates to nDerp1 and nDerp2.

The term "treating" means partly or wholly curing or alleviating symptoms, or inhibiting causes of symptoms.

The term "allergen" means any type of antigen that produces an abnormally vigorous immune response in which the immune system fights off a perceived threat that would otherwise be harmless to the body. The term "allergen" also means a protein or protein fragment that may elicit an allergic response in a patient sensitized to that allergen.

The term "allergy" means any type of hypersensitivity reaction to an environmental allergen mediated by immunological mechanisms, including type IV hypersensitivity reactions, including allergic rhinitis, asthma and atopic dermatitis.

The term "sensitized" means that a subject exhibits an IgE response specific to an allergen administered or encountered.

The term "crude" means that the HDM extract is not purified, representing the full array of major allergens present in house dust mite cultures.

### Allergen composition

The allergen composition of the invention comprises the natural form of Der p1 and Der p2 or Der f1 and Der f2 allergens in substantially pure form.

Generally, each of nDerp1 and nDerp2 have a purity of at least about 80 wt% (wt% on dry matter), preferably at least about 85 wt%, more preferably at least about 90 wt%, more preferably at least about 95 wt%, and even more preferably at least about 98% wt. The nDerp1 or nDerp2 may naturally contain one or more isoforms of the same allergen. The same applies to nDerf1 and nDerf2.

The purity of the allergen can be established via classical biochemical purification techniques.

In a preferred embodiment of the invention, the allergen composition is a purified allergen extract from house dust mites, comprising a mixture of purified nDerp1 and purified nDerp2. The same applies to nDerf1 and nDerf2.

The allergen composition contains as allergens 50 wt% or more nDerp1 and nDerp2, preferably about 80 wt% or more, and even more preferably about 90 wt% or more. The allergen composition may contain residual amounts of other proteins from HDM. The allergen composition may also contain other allergens, and preferably well-defined allergens. The same applies to nDerf1 and nDerf2.

In crude house dust mite extract, Der p1 proteins generally constitute about 0.2 - 2 wt% of the composition. Depending on the source, Der p2 generally constitutes about 0.1 to 1.5 times the amount of Der p1. The same applies to nDerf1 and nDerf2.

The nDerp1 and nDerp2 allergens may be present in the composition in equimolar amounts or the ratio of the allergens present may vary. The same applies to nDerf1 and nDerf2.

nDerp1 and nDerp2 are present in a weight ratio between 99:1 and 20:80, more preferably between about 95:5 and about 25:75. The same applies to nDerf1 and nDerf2.

In one preferred embodiment, the ration nDerp1 and nDerp2 is between about 95:5 and about 80:20, as that reflects the ratio in house dust mite excrements. The composition comprises for example nDerp1 and nDerp2 in a weight ratio of about 90:10. The same applies to nDerf1 and nDerf2.

In another embodiment, the ratio between nDerp1 and nDerp2 is between about 20:80 and 50:50, as that resembles the ratio of these proteins in the mite bodies. The composition comprises for example nDerp1 and nDerp2 in a weight ratio of about 35:65. The same applies to nDerf1 and nDerf2.

In a most preferred embodiment, nDerp1 is present in amounts larger than nDerp2. The same applies to nDerf1 and nDerf2.

Preparations of pharmaceutical compositions with proteins are generally known in the art and a skilled person will be able to prepare suitable compositions comprising nDerp1 and nDerp2. The same applies to nDerf1 and nDerf2.

Prior to administration the purified nDerp1 and nDerp2 may suitably be mixed with diluents and excipients, which are pharmaceutically acceptable and further compatible with the active ingredients. Examples of suitable diluents or excipients include water, saline, glycerol, ethanol, dextrose, lactose and the like as well as combinations thereof. The same applies to nDerf1 and nDerf2.

The compositions may additionally contain other substances such as wetting agents, emulsifying agents, buffering agents or adjuvants enhancing the immune reaction to the composition. In an embodiment of the invention nDerp1 and nDerp2 may be an allergoid wherein the allergens are complexed by for example glutaraldehyde or formaldehyde. The same applies to nDerf1 and nDerf2.

In an embodiment of the invention the purified nDerp1 and purified nDerp2 are dissolved in saline or PBS.

### Administration

In an embodiment of the invention the composition comprising purified nDerp1 and nDerp2 is administered in a manner selected from the group consisting of subcutaneous-, sublingual-, intradermal-, transdermal-, epicutaneous-, in the lymph nodes, oral- administrations or combinations thereof. The same applies to nDerf1 and nDerf2, and hereinbelow, the word nDerp1 and nDerp2 comprise nDerf1 and nDerf2, although Derp is preferably used.

In a preferred embodiment the composition comprising purified nDerp1 and nDerp2 is administered subcutaneously, transdermally or a combination thereof.

In a preferred embodiment the composition comprising purified nDerp1 and nDerp2 is administered subcutaneously.

The compositions with purified nDerp1 and nDerp2 are administered in a way so as to be compatible with the dosage formulation and in such an amount as will be therapeutically effective and immunogenic.

The quantity of nDerp1 and nDerp2 contained within the composition in a dosage form is generally between about 0.001 µg and about 1000 µg, preferably between about 0.01 µg and about 500 µg.

The amounts may vary largely, depending on a number of factors. These factors include, but are not limited to (i) patients, which may be very sensitive, or less sensitive to HDM, requiring different dosing (ii) by the dosage scheme, which itself may call for an increased dosing from very diluted to concentrated, over a factor of 100-1000; and (iii) by the way the composition is administered. Generally, sublingual administration uses 10-100 times higher doses than parenteral, although this is just a rule of the thumb.

In a preferred embodiment an administered SCIT or SLIT composition dose comprises about 1000 µg nDerp1, about 500 µg nDerp1, about 400 µg nDerp1, about 300 µg nDerp1, about 200 µg nDerp1, about 150 µg nDerp1, about 100 µg nDerp1, about 80 µg nDerp1, about 60 µg nDerp1, about 40 µg nDerp1, about 35 µg nDerp1, about 30 µg nDerp1, about 28 µg nDerp1, about 26 µg nDerp1, about 24 µg nDerp1, about 22 µg nDerp1, about 20 µg nDerp1, about 18 µg nDerp1, about 17 µg nDerp1, about 16 µg nDerp1, about 15 µg nDerp1, about 14 µg nDerp1, about 13 µg nDerp1, about 12 µg nDerp1, about 11 µg nDerp1, about 10 µg nDerp1, about 9.5 µg nDerp1, about 9,0 µg nDerp1, about 8,5 µg nDerp1, about 8,0 µg nDerp1, about 7,5 µg nDerp1, about 7,0 µg nDerp1, about 6,5 µg nDerp1, about 6,0 µg nDerp1, about 5,5 µg nDerp1, about 5,0 µg nDerp1, about 4,5 µg nDerp1, about 4,0 µg nDerp1, about 3,5 µg nDerp1, about 3,0 µg nDerp1, about 2,5 µg nDerp1, about 2,0 µg nDerp1, about 1,5 µg nDerp1, about 1,0 µg nDerp1, about 0,75 µg nDerp1, about 0,5 µg nDerp1, about 0,4 µg nDerp1, about 0,3 µg nDerp1, about 0,2 µg nDerp1 or about 0,1 µg nDerp1.

In another preferred embodiment an administered SCIT or SLIT composition dose comprises about 500 µg nDerp2, about 400 µg nDerp2, about 300 µg nDerp2, about 200 µg nDerp2, about 150 µg nDerp2, about 100 µg nDerp2, about 80 µg nDerp2, about 60 µg nDerp2, about 40 µg nDerp2, about 35 µg nDerp2, about 30 µg nDerp2, about 28 µg nDerp2, about 26 µg nDerp2, about 24 µg nDerp2, about 22 µg nDerp2, about 20 µg nDerp2, about 18 µg nDerp2, about 17 µg nDerp2, about 16 µg nDerp2, about 15 µg nDerp2, about 14 µg nDerp2, about 13 µg nDerp2, about 12 µg nDerp2, about 11 µg nDerp2, about 10 µg nDerp2, about 9.5 µg nDerp2, about 9,0 µg nDerp2, about 8,5 µg nDerp2, about 8,0 µg nDerp2, about 7,5 µg nDerp2, about 7,0 µg nDerp2, about 6,5 µg nDerp2, about 6,0 µg nDerp2, about 5,5 µg nDerp2, about 5,0 µg nDerp2, about 4,5 µg nDerp2 about 4,0 µg nDerp2, about 3,5 µg nDerp2, about 3,0 µg nDerp2, about 2,5 µg nDerp2, about 2,0 µg nDerp2, about 1,5 µg nDerp2, about 1,0 µg nDerp2, about 0,75 µg nDerp2, about 0,5 µg nDerp2, about 0,4 µg nDerp2, about 0,3 µg nDerp2, about 0,2 µg nDerp2 or about 0,1 µg nDerp2.

In an embodiment an administered SCIT or SLIT composition dose comprises nDerp1 and nDerp2 in a ratio of about 90:10 at a dose of about 1000 µg nDerpland about 111 µg nDerp2, or in a ratio of about 90:10 at a dose of about 300 µg nDerpland about 33 µg nDerp2, or in a ratio of about 90:10 at a dose of about 20 µg nDerpland about 2 µg nDerp2, or in a ratio of about 20:80 at a dose of about 200 µg nDerpland about 800 µg nDerp2, or in a ratio of about 20:80 at a dose of about 20 µg nDerpland about 80 µg nDerp2, or in a ratio of about 20:80 at a dose of about 100 µg nDerpland about 400 µg nDerp2, or in a ratio of about 99:1 at a dose of about 100 µg nDerpland about 1 µg nDerp2, or in a ratio of about 99:1 at a dose of about 1000 µg nDerpland about 10 µg nDerp2, or in a ratio of about 99:1 at a dose of about 10 µg nDerpland about 0,1 µg nDerp2.

In a preferred embodiment the composition comprising purified nDerp1 and purified nDerp2 is used in the treatment of a mammal suffering from HDM allergy.

In a preferred embodiment, the treatment of the mammal is allergen-specific immunotherapy (SIT). SIT involves administering doses of allergens to accustom the body to substances that are generally harmless (house dust mites) and thereby induce specific long-term tolerance.

For example, the SIT dosing regimen may comprise administering multiple doses of the composition to the subject suffering from HDM allergy at a frequency of about multiple times per day, once a day, once every two days, once every three days, once every four days, once every five days, once every six days, once a week, once every two weeks, once every three weeks, once every four weeks, once a month, once every two months, once every three months, once every four months, or less frequently. A treatment schedule may for example comprise first subcutaneously administering once a week a low dose of allergen (f.e. 10 µg) gradually increasing every week (f.e. from 20 µg to 100 µg) followed by monthly injections with for example 100 µg for a year and then once per 6 months.

In an embodiment of the invention a treatment schedule may comprise first up to 6 subcutaneous injections per day, followed by up to about 5 weekly injections, then weekly subcutaneous injections, then once every 2 weeks followed by monthly injections. In yet another embodiment of the invention a treatment schedule may comprise daily sublingual administration of drips.

The composition may also be provided in the form of a kit, containing purified nDerp1 and purified nDerp2 in separate dosage forms for simultaneous or consecutive administration.

As used herein, the expressions "allergen-specific immunotherapy" and "SIT" and the like, refer to the repeated administration of compositions comprising purified nDerp1 and nDerp2 to a subject over time as means for treating or preventing allergies and allergic reactions, or to reduce or eliminate allergic responses. In a typical SIT regimen, small amounts of allergen are initially administered to an allergic subject, followed by administration of increased amounts of allergen.

In certain instances, the SIT treatment may comprise at least two consecutive phases: (1) an up-dosing phase, and (2) a maintenance phase. In the up-dosing phase, increasing doses of purified nDerp1 and nDerp2 are administered until an effective and safe dose is achieved. The dose that is established at the end of the up-dosing phase is then administered to the subject throughout the course of the maintenance phase. The duration of the up-dosing phase can be several weeks or several months. In certain embodiments, however, the up-dosing phase is of substantially shorter duration (e.g., less than one week, less than 6 days, less than 5 days, less than 4 days, less than 3 days, or less than 2 days). The maintenance phase of an SIT regimen can last several weeks, several months, several years, or indefinitely.

In a preferred embodiment, the composition according the invention, comprising purified nDerp1 and purified nDerp2, is administered to a patient suffering from HDM allergy in an amount sufficient to reduce or abrogate IgE production and/or to reduce or eliminate serum IgE levels specific to house dust mite allergens, in particular to Der p1 and Der p2.

As used herein, a reduction in serum IgE level means that the amount of IgE measured in the serum of a subject who has been exposed to an allergen and who has been treated with purified nDerp1 and purified nDerp2, is at least 5%, 10%, 20%, 50%, 80%, or 90% lower than the serum IgE level measured in the same or an equivalent subject that has not been treated with the composition comprising purified nDerp1 and purified nDerp2.

### Subject treated

In a preferred embodiment of the invention, the mammal generally is a human or domestic animal or the like. Domestic animals that can be treated include cat, dog or horse. The preferred mammal is a human.

Generally, the human is a human patient that shows an allergic reaction to HDM.

In a preferred embodiment of the invention, the human patient suffering from HDM allergy suffers at least from one of the following allergic responses to HDM allergens: rhinitis, asthma and atopic dermatitis.

The human suffering from HDM allergy generally suffers from a reaction of HDM allergens with allergen specific IgE antibodies, release of histamine and other mediators (including but not limited to prostaglandins and leukotrienes) and tryptase from basophils and mast cells, eosinophilic lower and/or upper airway inflammation as well as other mucosal tissue and any other tissue in general, and airway hyperreactivity, release of pro-inflammatory cytokines and chemokines from type-2 innate lymphoid cells (ILC2s), Th-2 cells, basophils, mast cells and any other innate or adaptive immune cell, or combinations thereof upon HDM exposure.

House dust mite allergy plays an important role in asthma and allergic rhinitis and treatment of these groups is most preferred.

In an embodiment of the invention, treatment of a subject suffering from HDM allergy with a composition comprising purified nDerp1 and nDerp2 reduces or resolves reaction to exposure to house dust mites, i.e the subject is desensitized to the allergic response.

In a preferred embodiment treatment with purified nDerp1 and purified nDerp2 suppresses eosinophilic airway inflammation. The cell count of eosinophils present in broncho-alveolar lavage (BAL) fluid of a patient suffering from HDM allergy treated with purified nDerp1 and purified nDerp2 may decrease.

In a preferred embodiment treatment with purified nDerp1 and purified nDerp2 decreases the number of eosinophils in de BAL at least 3 about fold, more preferably about 5 fold, even more preferably about 7 fold.

In another embodiment of the invention treatment with a composition comprising purified nDerp1 and purified nDerp2 reduces or resolves airway hyperreactivity induced by house dust mite exposure by at least about 20%, preferably by about 30%, more preferably by about 40%, even more preferably by about 50%.

In yet another embodiment lung compliance may increase after treatment with purified natural Der p1 and Der p2. Preferably the lung compliance increases by about 5%, more preferably by about 15%, even more preferably by about 25%.

In an embodiment of the invention, SIT treatment of a patient suffering from HDM allergy with a composition comprising purified nDerp1 and purified nDerp2 suppresses Th-2 cell activity and induce Tr cell mediated tolerance. In an embodiment of the invention suppression of Th2 cell activity is characterized but not limited to reduced IL-4 and IL-5 cytokine production. In a further preferred embodiment the SIT treatment with nDerp1 and nDerp2 suppresses innate immune activity, characterized but not limited to suppressed CCL-20, CCL17 and/or eotaxin chemokine production upon exposure to HDM allergens. In a preferred embodiment SIT treatment with nDerp1 and nDerp2 suppresses the adaptive immune reaction. In yet another preferred embodiment SIT treatment with nDerp1 and nDerp2 suppresses activity of both the adaptive and the innate immune response.

In another embodiment of the invention IL-13, eotaxin, CCL17 and IL-33 may be suppressed after treatment with the composition comprising purified nDerp1 and purified nDerp2.

In yet another embodiment of the invention lung dendritic cell phenotype may change after treatment with the composition comprising purified nDerp1 and purified nDerp2.

In a preferred embodiment the phenotype of the lung dendritic cells becomes more tolerogenic.

In an embodiment of the invention inflammatory or monocyte-derived dendritic cells (DCs) may be suppressed after treatment with the composition comprising purified nDerp1 and purified nDerp2, whereas CD103⁺ or any other class of tolerogenic DCs may increase.

### Preparation of allergen

The isolation of nDerp1 and nDerp2 is described in the literature since Chapman et al., J. Immunol. 1984; 125(2):587-593, although the processes are cumbersome, and large amounts of purified nDerp1 are not easy to obtain.

The purification of allergens from HDM (Der p as well as Der f) can be performed from mite extracts according to the methods described in Bordas - Le Floch et al. Int. Arch. Allergy Immunol. 2012; 158:157-167; Yasueda et al. Int. Archs. Allergy Appl. Immun. 1986; 214-223 and de Halleux et al. J. Allergy clin. Immunol. 2006; 571-576 by ion exchange chromatography.

Der p 1 may be purified using a SEC HiPrep 26/60 Sephacryl S-300 column with 50 mM NH₄HCO₃ as buffer. The same technology may be used for the isolation of Der f1.

Der p2 may be purified using a phenyl sepharose column (GE Healthcare, Saclay, France) with 20 mM sodium phosphate, 1.8 M ammonium sulphate pH 6.2, as well as 20 mM sodium phosphate, 5% isopropanol pH 7.5 as binding and elution buffers, respectively.

Der p2 may be purified using cationic exchange on SP-Sepharose column with 25 mM sodium acetate as binding buffer and a 0% to 100% linear gradient to 1 M NaCl as elution buffer. Further purification may be performed by affinity on a benzamidine column with 50 mM Tris, 0.5 M NaCl, pH7.5 as binding buffer, using a 0% to 100% linear gradient to 100 mM glycine buffer pH 2.7 for elution. The same technology may be used for the isolation of Der f2.

nDerp2 as well as nDerf2 may be obtained recombinantly, providing that the natural structure is obtained and preserved.

The several purification/isolation steps are preferably performed at low temperatures, preferable lower than about 10 °C, like for example around 4 °C. Not being bound by theory it is postulated that isolation of Der p1 and Der p2 performed at low temperature limits proteolytic activity and autoproteolysis of Der p1.

Isolated, purified nDerp1 and nDerp2 are in a preferred embodiment freeze dried and stored.

The composition of the invention may be stored in dry powdered form in vials suitable for reconstitution of for example injectable liquid. The composition of the invention may be formulated in suitable dosage forms as known by the skilled person and as described above.

The invention is further described below by way of examples with reference to the following experimental procedures and results.

### EXAMPLES

In summary, the inventors of this application used purified nDerp1 and nDerp2 (>95 wt% pure) for SCIT of HDM allergy. Purified nDerp1 and nDerp2 as well as crude HDM extract were used in subcutaneous immunotherapy (SCIT) in a mouse model of HDM driven allergic asthma. Both HDM-SCIT and nDerp1/nDerp2-SCIT were able to suppress airway hyperresponsiveness (AHR) and eosinophilic airway inflammation.

The composition comprising nDerp1- and nDerp2-SCIT used in the treatment was found to uniquely suppress type-2 cytokine production in the lungs as well as CCL20 production, indicating a suppression of both the adaptive Th2 response as well as the innate (ILC2 and airway epithelial) immune response. SCIT treatment with the composition comprising purified nDerp1 and nDerp2 was therefore more effective than crude HDM extract.

In one embodiment, the present invention is therefore specifically directed purified natural Der p1 and Der p2 for use to suppress Th2 cytokine production caused by house dust mite allergy.

In another embodiment, the present invention is therefore (also) specifically directed to purified natural Der p1 and Der p2 for use to suppress of CCL20 production caused by house dust mite allergy.

### Example 1 - Assessment of the induction house dust mite neutralizing antibodies

Mice used in the following illustrative examples of house dust mite driven allergic asthma were female BALB/cByJ mice (7-8 weeks of age) purchased from Charles River Laboratories (L'Arbresle Cedex, France). Mice were kept in individually ventilated cages, maintained on a 12 hours light dark cycle, with standard food and water *ad libitum.* No mice died during the experiments.

All mice received two sensitizing intraperitoneal injections of 100 µL PBS containing 5 µg crude house dust mite (HDM) extract (Citeq Biologics, Groningen, The Netherlands) adsorbed to 2.25 mg Alum (Pierce, USA).

All experimental procedures were in accordance with the guidelines of and approved by the local ethical committee on animal welfare.

To assess the induction of a neutralizing antibody response by SIT treatment, HDM sensitized mice underwent SCIT treatment. SCIT was performed by three subcutaneous injections containing different formulations of 100 µl injected every other day in one week. In mice, IgG1 and IgG2a characterize a neutralizing antibody response. All but the NC group was HDM challenged.

HDM sensitized mice were divided in groups of n=8 mice that were SCIT treated with:
1) Phosphate buffered saline (PBS), PBS-SCIT and PBS challenged (NC, sensitized only),
2) Phosphate buffered saline (PBS), PBS-SCIT and HDM challenged (PC, full blown allergic asthma),
3) Lipopolysaccharide group (LPS-SCIT challenged with HDM, 145 µg LPS in 100 µl PBS),
4) Full/ crude house dust mite (HDM) body extract (HDM-SCIT, 250 µg HDM protein in 100 µl PBS),
5) Purified nDerp1 and nDerp2 (DER-SCIT); the ratio of nDerp1 to nDerp2 in the composition was 90:10 (wt/wt) with 100 µg Derp1/p2 protein in µl PBS.
ELISA was performed to determine the concentration of IgG₁ (Bethyl Laboratories, Inc., Montgomery, TX, USA), IgG_{2A}(Bethyl Laboratories, Inc., Montgomery, TX, USA), and IgE (BioLegend, San Diego, CA, USA) in serum taken at three different time points (pre1-, pre2-, and post-serum). Pre1- and pre2-serum was obtained through orbital puncture, whilst post-serum was obtained through vena puncture during section. All serum samples were collected in MiniCollect tubes (Greiner Bio-One LTD., Gloucestershire, United Kingdom) and centrifuged for 11 minutes at 18000g. Samples were then stored at -20°C until further use. IgE, IgG₁ and IgG₂ₐ immunoglobulin levels in serum were measured before and after the SCIT treatment as illustrated in Figure 1. The induction of IgG₁ and IgG₂ₐ after SCIT treatment was significant in the DER-SCIT groups (figure 1).
SCIT treatment using nDerp1 and nDerp2 allergens was superior to SCIT treatment using HDM extract in inducing HDM-specific neutralizing IgG2a antibodies in serum of HDM-sensitized mice. HDM-SCIT treatment did not induce HDM specific IgG2a antibodies in serum of HDM-sensitized mice.

### Example 2 - Assessment of the protection against IgE- mediated response to HDM challenge after SCIT treatment

To assess the protection against early IgE mediated responses upon house dust mite re-challenge in the mice described in example 1, mice underwent an ear-swelling test (EST) by intradermal injection of HDM extract one week prior to SIT treatments (PreSCIT) and two days before the challenges (PostSCIT). Herein, 0,5 µg crude extract HDM in 10 µL PBS is injected intradermal in the right ear of anesthetized mice, while 10 µL of PBS is injected in the left as a control (REF). After two hours, ear thickness was measured using a digimatic force-micrometer at 0.5N (± 0.15N, Mitutoyo, Japan). The net fold change in ear thickness (Δ, in mm) was calculated by dividing the thickness of the left ear over the thickness of the right ear.

Injection of the allergen extract in HDM sensitized and PBS-control treated mice resulted in a significant ear swelling response as illustrated in Figure 2, indicative of the presence of an IgE mediated early response to allergen re-challenge. Ear swelling response was significantly reduced by about 50% in HDM-SCIT treated mice (p<0.05 in Mann whitney U test). Ear swelling response was reduced (not significantly) in DER-SCIT treated mice.

### Example 3 - Assessment of lung function and allergic airway inflammation

The mice from example 1 and 2 subsequently underwent HDM inhalation challenges to assess lung function parameters and allergic airway inflammation. Inhalation challenges were performed using 25 µg crude extract HDM dissolved in 25 µL PBS, given three times over a period of 5 days. Two days thereafter, airway responsiveness to methacholine was determined, and serum samples, broncho-alveolar lavage fluid (BALF), and lung lobes were stored for further analyses.

Airway responsiveness was assessed by measuring airway resistance in response to intravenous administration of increasing doses of methacholine (Sigma-Aldrich, MO) using a computer-controlled small-animal ventilator (FlexiVent; SCIREQ, Montreal, Quebec, Canada) .

Airway resistance R was measured in cmH2OL.s/ml in response to a dose-range of methacholine from 0 to 800 µg/kg body weight in the groups of HDM sensitized, SCIT treated and subsequently HDM or PBS challenged mice. In short, mice were injected intraperitoneal with a mixture of 100 mg/kg ketamine (Pfizer, NY) and 1 mg/kg dormitor (Pfizer), and intravenously with 1.875 mg/kg body weight Rocuronium Bromide (administered both at the start of the experiment and after the 100 methacholine dose). After tracheotomization using a 20-gauge intravenous cannula (Becton Dickinson, The Netherlands) and canalization through the jugular vein, all mice were attached to a FlexiVent and then ventilated (280 breaths/minute) with a tidal volume of 10 mL/kg, pressure limited at 300 mmH₂O. In response to increasing dosages of methacholine, the airway resistance was calculated from the pressure response to a 2-second pseudorandom pressure wave (COD > 0.75).

As illustrated in figure 3 HDM-SCIT and DER-SCIT both significantly reduced the airway hyperresponsiveness in the HDM challenged mice as evidenced by the right shift of the dose-response curve and by a reduced plateau level of the airway resistance with increasing methacholine concentrations. Strongest suppression of airway hyperresponsiveness to methacholinewas observed in the DER-SCIT mice with effective suppression at a dosis of 20 and 100 microgram per injection of methacholine.

The mice that were HDM sensitized and underwent PBS control treatment had highest airway resistance upon intranasal HDM challenge illustrative of allergic asthmatic response, whereas the mice that were challenged with PBS had lowest airway resistance.

In addition to comparing the methacholine dose-response curves between the experimental groups, the dose of methacholine required for an airway hyperresponsiveness of 3 cmH₂O.s/ml (Effective Dose, ED3) was calculated. ED3 is an indication for the slope of the dose-response curve at lower methacholine concentrations. Both HDM-SCIT and Der-SCIT treated mice show a trend towards an increased ED3 as compared to the positive control group of asthmatic mice (0.05<p<0.1), indicative of a lower airway hyperresponsiveness (figure 4).

In conclusion, SCIT treatment with nDerp1 and nDerp2 allergens is superior to SCIT treatment using HDM extract in suppressing airway hyperresponsiveness to methacholine

### Example 4 - Assessment of lung compliance

Lung compliance is a measure of the lung's ability to stretch and expand whereby low compliance indicates a stiff lung and high compliance indicates a difficulty exhaling air.

Lung compliance (C) in ml/cmH2O was assessed in all experimental groups described above in response to the increasing doses of methacholine. A significantly reduced lung compliance was observed in all HDM challenged groups as illustrated in Figure 5. HDM-SCIT and Der-SCIT did not increase lung compliance.

### Example 5 - assessment of SCIT treatment on eosinophilic airway inflammation

Desensitization to allergens is associated with suppression in airway inflammation upon allergen (re)challenge.

The eosinophilic airway inflammation profile was evaluated in broncho-alveolar lavage fluid in HDM sensitized mice as described in the previous examples. The lungs were lavaged with 1 mL PBS containing 5% BSA and a cocktail of protease inhibitors (Complete mini tablet; Roche, Germany). Subsequently, using 4 mL PBS, the lungs were lavaged for the collection of extra cells. The first mL broncho-alveolar lavage fluid (BALF) was stored in duplicate at -80°C for cytokine analysis (example 6-7). All BALF samples as well as the lung single cell suspensions were counted using the Coulter Counter. Cytospin preparations of both sample types were stained with Diff-Quick (Merz & Dade, Switzerland) and 300 cells per cytospin were evaluated by one experienced technician and differentiated into mononuclear cells (M), neutrophils (N), and eosinophils (E) by standard morphology.

HDM challenges induced a pronounced eosinophilic airway inflammation in HDM sensitized mice, with presence of neutrophils (N) as illustrated in figure 6. HDM-SCIT treatment and DER-SCIT treatment suppressed the absolute numbers of eosinophils (E) in BAL fluid by about 5-fold as compared to PBS-SCIT treated HDM challenged mice, while the numbers of neutrophils in BAL were also suppressed.

### Example 6 - assessment of the effect of SCIT treatment on Thelper-2 cell activity

Desensitization to allergens with allergen immunotherapy is thought to suppress activity of allergen-specific Th-2 cells and induce regulatory T cell mediated tolerance to allergens.

HDM sensitized and subsequently control treated or SCIT treated mice as described in the previous examples were sacrificed and long tissue was isolated. To relate the amount of cytokines measured with Luminex assays and ELISA to total protein content, a BCA protein assay (ThermoScientific, Thermo Fisher Scientific Inc., Waltham, MA, US; ref 23 227) was performed. One lung lobe from each mouse was weighted and Luminex buffer with Photostop (F. Hoffmann-La Roche Ltd., Basel, Switserland; Ref 04906845001) was added in a ratio of 1:5. Lobes were homogenized with a homogenizer and homogenized lungs were centrifuged for 20 minutes at 12,000g. Supernatants were collected and devided into aliquots at -80°C until further use, a BCA analysis was performed according to the manufacturer's protocol.

Interleukin-4 (IL-4), IL-5, IL-13, Interferon-gamma (IFN-y) and IL-17 T-cell cytokines in pg/pg lung tissue were evaluated for the different treatment groups using a multiplex Mouse Cytokine/Chemokine Magnetic Bead Panel (MILLIPLEX Map Kit; Merck Millipore Corp., Merck KGaA, Darmstadt, Germany; Cat. #MCYTOMAG-70K-10) according to manufacturer's protocol. All plates were analysed using a MAGPX1023 4002 with Luminex xMAP technology.

As illustrated in figure 7, HDM challenge in HDM sensitized and PBS-SCIT treated mice (PC) clearly induced IL-4, IL-15 and IL-13, as well as IL-17 in lung tissue compared to PBS challenged PBS-SCIT control treated and HDM sensitized mice (NC). DER-SCIT treatment, but not HDM-SCIT significantly suppressed the HDM-induced IL-4 and IL-5 production, while the effects on IL-13 were not as strong in either of the 2 groups. Interestingly, IFN-y was not induced by HDM challenge or SCIT treatment in this model while IL-17 was clearly induced by HDM challenges, but not significantly suppressed by either HDM-SCIT or DER-SCIT treatment, indicating that the treatment preferentially acted on Th2 cells or innate type-2 helper cells.

In addition to evaluating the T cell cytokines, the effect of SCIT treatment on chemokines and alarmins released by the lung structural cells was also assessed in pg/pg lung tissue.

Concentrations of TARC (CCL17) and MIP-3α (CCL20) were measured using a multiplex Mouse Cytokine/Chemokine Magnetic Bead Panel II (MILLIPLEX Map Kit; Merck Millipore Corp., Merck KGaA, Darmstadt, Germany; Cat. #MCYP2MAG-73K) according to manufacturer's protocol. IL-33 concentrations were measured using a multiplex Mouse Cytokine/Chemokine Magnetic Bead Panel III (MILLIPLEX Map Kit; Merck Millipore Corp., Merck KGaA, Darmstadt, Germany; Cat. #MCYP3MAG-74K) according to manufacturer's protocol. Concentrations of eotaxin and KC were measured using a multiplex Mouse Cytokine/Chemokine Magnetic Bead Panel (MILLIPLEX Map Kit; Merck Millipore Corp., Merck KGaA, Darmstadt, Germany; Cat. #MCYTOMAG-70K-10) according to manufacturer's protocol. All plates were analysed using a MAGPX1023 4002 with Luminex xMAP technology.

HDM challenge in HDM sensitized mice clearly induced release of eotaxin, TARC/CCL17, MIP3a/CCL20, IL-33 and KC/CXCL1, indicating pro-inflammatory activation of the innate immune system and lung structural cells as illustrated in figure 8. Interestingly, a trend towards suppression of the release of eotaxin, CCL17 and IL-33 after DER-SCIT treatment, while production of CCL20 was significantly suppressed. No significant effect of DER-SCIT treatment on KC release was observed.

Overall, SCIT treatment using nDerp1 and nDerp2 allergens was superior to SCIT treatment using HDM extract in suppressing the production of IL-4, IL-5, CCL17, CCL20 and eotaxin-1 in HDM-sensitized and HDM-challenged mice. nDerp1 and nDerp2 SCIT treatment has been found to suppress activation of both the innate and the adaptive immune response upon house dust mite challenges in a sensitized host. The mechanism of nDerP1/2 SCIT mediated suppression of allergic inflammation and airway hyperresponsiveness in HDM-sensitized and HDM-challenged mice involved suppression of the activation of adaptive Th2 cells as well as the innate immune system.

## Claims

1. Pharmaceutical composition for use in the treatment of a mammal suffering from house dust mite allergy, said composition comprising an allergen composition in a pharmaceutically acceptable diluent or excipient, said allergen composition comprising purified natural *Dermatophagoides pteronyssinus* (Der p)1 and purified natural Der p2, or purified natural *Dermatophagoides farinae* (Der f)1 and purified natural Der f2, wherein the allergen composition contains 50 wt%, on dry matter, or more of natural Der p1 and natural Der p2, or natural Der f1 and natural Der f2 allergens, wherein the allergen composition comprises purified natural Derp1 and purified natural Derp2, or purified natural Der f1 and purified natural Der f2 in a ratio of 20:80 to 99:1, and wherein the treatment with purified natural Der p1 and purified natural Der p2, or purified natural Der f1 and purified natural Der f2 desensitizes the patient's allergic response to house dust mite, the term "natural" meaning that the chemical structure of the protein is identical to the natural occurring protein in its primary, secondary and tertiary structure.

2. Composition for use according to the preceding claim, wherein the mammal is a human being.

3. Composition for use according to any of the preceding claims wherein the mammal suffering from house dust mite allergy suffers at least from one of the following allergic responses to house dust mite allergen: rhinitis, asthma and atopic dermatitis.

4. Composition for use according to any one of the preceding claims, wherein the desensitization is **characterized by** suppression of the Th2 and/or ILC2 cell activity upon house dust mite exposure.

5. Composition for use according to the preceding claim, wherein the suppression of the Th2 and/or ILC2 cell activity is **characterized by** a reduction in IL-4 and IL-5 cytokine production.

6. Composition for use according to any of the preceding claims wherein the desensitization is **characterized by** suppression of the CCL20 release upon house dust mite exposure, wherein preferably the CCL20 release is suppressed by at least 1 fold relative to release prior to treatment.

7. Composition for use according to any of the preceding claims wherein desensitization is **characterized by** the reduction of eosinophilic airway inflammation or airway hyperreactivity induced by house dust mite exposure.

8. Composition for use according to any one of the preceding claims, wherein the allergen composition comprises purified natural Derp1 and purified natural Derp2, or purified natural Der f1 and purified natural Der f2, wherein each of said allergens have been isolated with a purity of at least 80 wt%, preferably at least 90%, and combined in the allergen composition of claim 1.

9. Composition for use according to any one of the preceding claims, wherein the allergen composition comprises purified natural Derp1 and purified natural Derp2, or purified natural Der f1 and purified natural Der f2 in a ratio of 25:75 to 95:5.

10. Composition for use according to any one of the preceding claims, wherein the allergen composition contains as allergens about 80 wt% or more of natural Der p1 and natural Der p2, or natural Der f1 and natural Der f2.

11. Composition for use according to any of the preceding claims wherein the composition comprises 0.001-1000 microgram purified natural Der p1 and purified natural Der p2, or purified natural Der f1 and purified natural Der f2.

12. Composition for use according to any one of the preceding claims wherein the composition comprising purified natural Der p1 and purified natural Der p2, or purified natural Der f1 and purified natural Der f2 is administered in a manner selected from the group consisting of subcutaneously, sublingually, intradermally, transdermally, epicutaneously, orally, intralymphatically or combinations thereof.

13. Composition for use according to the preceding claim, wherein the purified natural Der p1 and purified natural Der p2, or purified natural Der f1 and purified natural Der f2 are administered subcutaneously, orally or sublingually.

14. Composition for use according to any of the preceding claims wherein the composition is administered repeatedly according to a specific immunotherapy regimen one to several times daily, weekly, monthly and/or yearly, with increasing dosages during the initial phase of the treatment.

15. Composition for use according to any one of the preceding claims, wherein the composition is in the form of a kit, containing purified natural Der p1 and purified natural Der p2, or purified natural Der f1 and purified natural Der f2 in separate dosage forms for simultaneous or consecutive administration.

## Patentansprüche

1. Pharmazeutische Zusammensetzung zur Verwendung bei der Behandlung eines Säugetiers, das an einer Hausstaubmilbenallergie leidet, die Zusammensetzung umfassend eine Allergenzusammensetzung in einem pharmazeutisch verträglichen Verdünnungsmittel oder einem Trägerstoff, die Allergenzusammensetzung umfassend gereinigte natürliche *Dermatophagoides pteronyssinus* (Der p)1 und gereinigte natürliche Der p2 oder gereinigte natürliche *Dermatophagoides farinae* (Der f)1 und gereinigte natürliche Der f2, wobei die Allergenzusammensetzung 50 Gew.-%, an einer Trockenmasse, oder mehr von natürlichen Der p1 und natürlichen Der p2 enthält,
oder natürliche Der f1- und natürliche f2-Allergene, wobei die Allergenzusammensetzung gereinigte natürliche Derp1 und gereinigte natürliche Derp2,
oder gereinigte natürliche Der f1 und gereinigte natürliche Der f2 in einem Verhältnis von 20 : 80 bis 99 : 1 umfasst, und wobei die Behandlung mit gereinigten natürlichen Der p1 und gereinigten natürlichen Der p2, oder gereinigten natürlichen Der f1 und gereinigten natürlichen Der f2 die allergische Reaktion des Patienten auf die Hausstaubmilbe desensibilisiert, wobei der Begriff "natürlich", bedeutet, dass die chemische Struktur des Proteins mit dem natürlich vorkommenden Protein in seiner primären, sekundären und tertiären Struktur identisch ist.

2. Zusammensetzung zur Verwendung nach dem vorstehenden Anspruch, wobei das Säugetier ein Mensch ist.

3. Zusammensetzung zur Verwendung nach einem der vorstehenden Ansprüche, wobei das Säugetier, das an der Hausstaubmilbenallergie leidet, mindestens an einer der folgenden allergischen Reaktionen auf ein Hausstaubmilbenallergen leidet: Rhinitis, Asthma und atopische Dermatitis.

4. Zusammensetzung zur Verwendung nach einem der vorstehenden Ansprüche, wobei die Desensibilisierung **durch** eine Unterdrückung der Th2- und/oder ILC2-Zellaktivität bei einer Hausstaubmilbenaussetzung **gekennzeichnet** ist.

5. Zusammensetzung zur Verwendung nach dem vorstehenden Anspruch, wobei die Unterdrückung der Th2 und/oder ILC2-Zellaktivität **durch** eine Verringerung der IL-4- und IL-5-Zytokinproduktion **gekennzeichnet** ist.

6. Zusammensetzung zur Verwendung nach einem der vorstehenden Ansprüche, wobei die Desensibilisierung **durch** die Unterdrückung der CCL20-Freisetzung bei einer Hausstaubmilbenaussetzung **gekennzeichnet** ist, wobei vorzugsweise die CCL20-Freisetzung um mindestens das 1-fache relativ zu der Freisetzung vor der Behandlung unterdrückt wird.

7. Zusammensetzung zur Verwendung nach einem der vorstehenden Ansprüche, wobei die Desensibilisierung **durch** die Verringerung einer eosinophilen Atemwegsentzündung oder einer Atemwegshyperreaktivität, die durch die Hausstaubmilbenaussetzung induziert wird, **gekennzeichnet** ist.

8. Zusammensetzung zur Verwendung nach einem der vorstehenden Ansprüche, wobei die Allergenzusammensetzung gereinigte natürliche Derp1 und gereinigte natürliche Derp2,
oder gereinigte natürliche Der f1 und gereinigte natürliche Der f2 umfasst, wobei jedes der Allergene mit einer Reinheit von mindestens 80 Gew.-%, vorzugsweise mindestens 90 %, isoliert wurde und in der Allergenzusammensetzung nach Anspruch 1 kombiniert wird.

9. Zusammensetzung zur Verwendung nach einem der vorstehenden Ansprüche, wobei die Allergenzusammensetzung gereinigte natürliche Derp1 und gereinigte natürliche Derp2,
oder gereinigte natürliche Der f1 und gereinigte natürliche Der f2 in einem Verhältnis von 25 : 75 bis 95 : 5 umfasst.

10. Zusammensetzung zur Verwendung nach einem der vorstehenden Ansprüche, wobei die Allergenzusammensetzung als Allergene etwa 80 Gew.-% oder mehr von natürlichen Der p1 und natürlichen Der p2, oder natürlichen Der f1 und natürlichen Der f2 enthält.

11. Zusammensetzung zur Verwendung nach einem der vorstehenden Ansprüche, wobei die Zusammensetzung 0,001 bis 1000 Mikrogramm gereinigte natürliche Der p1 und gereinigte natürliche Der p2, oder gereinigte natürliche Der f1 und gereinigte natürliche Der f2 umfasst.

12. Zusammensetzung zur Verwendung nach einem der vorstehenden Ansprüche, wobei die Zusammensetzung, umfassend gereinigte natürliche Der p1 und gereinigte natürliche Der p2, oder gereinigte natürliche Der f1 und gereinigte natürliche Der f2, in einer Weise verabreicht wird, die aus der Gruppe ausgewählt ist, bestehend aus subkutan, sublingual, intradermal, transdermal, epikutan, oral, intralymphatisch oder Kombinationen davon.

13. Zusammensetzung zur Verwendung nach dem vorstehenden Anspruch, wobei die gereinigten natürlichen Der p1 und gereinigten natürlichen Der p2, oder gereinigten natürlichen Der f1 und gereinigten natürlichen Der f2 subkutan, oral oder sublingual verabreicht werden.

14. Zusammensetzung zur Verwendung nach einem der vorstehenden Ansprüche, wobei die Zusammensetzung gemäß einem spezifischen Immuntherapieschema ein bis mehrmals täglich, wöchentlich, monatlich und/oder jährlich, mit steigenden Dosierungen während der Anfangsphase der Behandlung, wiederholt verabreicht wird.

15. Zusammensetzung zur Verwendung nach einem der vorstehenden Ansprüche, wobei die Zusammensetzung in der Form eines Kits ist, das gereinigte natürliche Der p1 und gereinigte natürliche Der p2, oder gereinigte natürliche Der f1 oder gereinigte natürliche Der f2 in separaten Dosierungsformen für eine gleichzeitige oder aufeinander folgende Verabreichung enthält.

## Revendications

1. Composition pharmaceutique utilisable pour le traitement d'un mammifère souffrant d'allergie aux acariens de la poussière de maison, ladite composition comprenant une composition d'allergènes dans un diluant ou excipient pharmaceutiquement acceptable, ladite composition d'allergènes comprenant *Dermatophagoides pteronyssinus* (Der p)1 naturel purifié et Der p2 naturel purifié, ou *Dermatophagoides farinae* (Der f)1 naturel purifié et Der f2 naturel purifié, dans laquelle la composition d'allergènes contient 50 % en poids, sur la matière sèche, ou plus d'allergènes Der p1 naturel et Der p2 naturel, ou Der f1 naturel et Der f2 naturel, dans laquelle la composition d'allergènes comprend Derp1 naturel purifié et Derp2 naturel purifié,
ou Der f1 naturel purifié et Der f2 naturel purifié en un rapport de 20:80 à 99:1, et dans laquelle le traitement avec Der p1 naturel purifié et Der p2 naturel purifié, ou Der f1 naturel purifié et Der f2 naturel purifié désensibilise la réponse allergique du patient à des acariens de la poussière de maison, le terme « naturel » signifiant que la structure chimique de la protéine est identique à la protéine d'origine naturelle concernant sa structure primaire, secondaire et tertiaire.

2. Composition utilisable selon la revendication précédente, dans laquelle le mammifère est un être humain.

3. Composition utilisable selon l'une quelconque des revendications précédentes, dans laquelle le mammifère souffrant d'allergie aux acariens de la poussière de maison souffre d'au moins l'une des réponses allergiques suivantes à un allergène d'acarien de la poussière de maison : rhinite, asthme et dermatite atopique.

4. Composition utilisable selon l'une quelconque des revendications précédentes, dans laquelle la désensibilisation est **caractérisée par** la suppression de l'activité de cellules Th2 et/ou ILC2 lors de l'exposition à des acariens de la poussière de maison.

5. Composition utilisable selon la revendication précédente, dans laquelle la suppression de l'activité de cellules Th2 et/ou ILC2 est **caractérisée par** une réduction de la production de cytokines IL-4 et IL-5.

6. Composition utilisable selon l'une quelconque des revendications précédentes, dans laquelle la désensibilisation est **caractérisée par** la suppression de la libération de CCL20 lors de l'exposition à des acariens de la poussière de maison, de préférence la libération de CCL20 étant supprimée d'un facteur d'au moins 1 par rapport à la libération avant traitement.

7. Composition utilisable selon l'une quelconque des revendications précédentes, dans laquelle la désensibilisation est **caractérisée par** la réduction d'inflammation à éosinophiles des voies respiratoires ou d'hyperréactivité des voies respiratoires induites par l'exposition à des acariens de la poussière de maison.

8. Composition utilisable selon l'une quelconque des revendications précédentes, dans laquelle la composition d'allergènes comprend Derp1 naturel purifié et Derp2 naturel purifié,
ou Der f1 naturel purifié et Der f2 naturel purifié, dans laquelle chacun desdits allergènes a été isolé avec une pureté d'au moins 80 % en poids, de préférence d'au moins 90 %, et combiné dans la composition d'allergènes selon la revendication 1.

9. Composition utilisable selon l'une quelconque des revendications précédentes, dans laquelle la composition d'allergènes comprend Derp1 naturel purifié et Derp2 naturel purifié,
ou Der f1 naturel purifié et Der f2 naturel purifié en un rapport de 25:75 à 95:5.

10. Composition utilisable selon l'une quelconque des revendications précédentes, dans laquelle la composition d'allergènes contient en guise d'allergènes environ 80 % en poids ou plus de Der p1 naturel et de Der p2 naturel, ou de Der f1 naturel et de Der f2 naturel.

11. Composition utilisable selon l'une quelconque des revendications précédentes, dans laquelle la composition comprend 0,001 à 1000 microgrammes de Der p1 naturel purifié et de Der p2 naturel purifié, ou de Der f1 naturel purifié et de Der f2 naturel purifié.

12. Composition utilisable selon l'une quelconque des revendications précédentes, dans laquelle la composition comprenant Der p1 naturel purifié et Der p2 naturel purifié, ou Der f1 naturel purifié et Der f2 naturel purifié est administrée d'une manière choisie dans le groupe constitué de voie sous-cutanée, voie sublinguale, voie intradermique, voie transdermique, voie épicutanée, voie orale, voie intralymphatique ou combinaisons de celles-ci.

13. Composition utilisable selon la revendication précédente, dans laquelle le Der p1 naturel purifié et le Der p2 naturel purifié, ou le Der f1 naturel purifié et le Der f2 naturel purifié sont administrés par voie sous-cutanée, par voie orale ou par voie sublinguale.

14. Composition utilisable selon l'une quelconque des revendications précédentes, dans laquelle la composition est administrée de manière répétée selon une posologie d'immunothérapie spécifique une à plusieurs fois par jour, par semaine, par mois et/ou par an, avec des doses en augmentation pendant la phase initiale du traitement.

15. Composition utilisable selon l'une quelconque des revendications précédentes, dans laquelle la composition est sous la forme d'un kit, contenant Der p1 naturel purifié et Der p2 naturel purifié, ou Der f1 naturel purifié et Der f2 naturel purifié dans des formes galéniques séparées pour administration simultanée ou consécutive.
